# EUROPÄISCHE PATENTSCHRIFT

(11) **EP 2 858 585 B1**
(45) Veröffentlichungstag und Bekanntmachung des Hinweises auf die Patenterteilung: **24.05.2017**
(21) Anmeldenummer: 13736472.5
(22) Anmeldetag: 11.06.2013
(51) Int. Cl.: A61B 17/86

(54) **KNOCHENSCHRAUBENANORDNUNG VARIABLER LÄNGE**
BONE SCREW ARRANGEMENT WITH VARIABLE LENGTH
ENSEMBLE DE VIS À OS DE LONGUEUR VARIABLE

(30) Priorität: 11.06.2012 DE 202012005594 U
(43) Veröffentlichungstag der Anmeldung: 15.04.2015
(73) Patentinhaber: Aristotech Industries GmbH, 12247 Berlin (DE)
(72) Erfinder: ANAPLIOTIS, Emmanuel, 14195 Berlin (DE)
(74) Vertreter: Bittner, Thomas L.
(86) Internationale Anmeldenummer: PCT/DE2013/100209
(87) Internationale Veröffentlichungsnummer: WO 2013/185755

(56) Entgegenhaltungen:
- WO-A1-89/06940
- DE-A1-102005 007 674
- US-A1- 2005 245 933
- US-A1- 2010 268 285

## Beschreibung

Die Erfindung betrifft eine Knochenschraubenanordnung variabler Länge.

### Hintergrund

Derartige Knochenschraubenanordnungen dienen zum Fixieren von Knochen oder Knochenstücken, insbesondere im Fall von Knochenfrakturen, derart, dass sich die Knochenschraubenanordnung dynamisch an sich ändernde Bedingungen im Heilungsprozess anpasst, indem sie ihre Länge verändert. Beispielsweise ist der Einsatz zur Behandlung der Epiphysiolysis capitis femoris (ECF) vorgesehen.

Eine Knochenschraubenanordnung variabler Länge ist aus dem Dokument WO 89/06940 A1 bekannt. Bei der bekannten Anordnung ist eine als Knochenschraube ausgeführte Verankerungsschraube vorgesehen, die in einer Führung, welche im Inneren einer Führungsschraube gebildet ist, axial verlagerbar aufgenommen ist. Auf diese Weise ist es ermöglicht, dass die Knochenschraubenanordnung ihre Gesamtschraubenlänge verändert, wenn die Knochenschraubenanordnung im menschlichen oder tierischen Körper angeordnet ist, beispielsweise aufgrund eines Knochenwachstums. Bei der Gesamtlängenveränderung ist ein Führungselement, welches kopfseitig an der Ankerschraube gebildet ist, in der Führung in Axialrichtung geführt. Das an der Ankerschraube gebildete Führungselement ist mit einem Sechskantkopf gebildet, der formschlüssig in der Führung an der Führungsschraube aufgenommen ist, sodass sich beim Drehen der Führungsschraube die Ankerschraube zwangsweise mitdreht.

Das Dokument DE 10 2005 007 674 A1 bezieht sich auf ein orthopädisches Fixiersystem. Bei der Verwendung des Systems wird in eine Knochplatte eine Traghülse eingeschraubt. Die Traghülse nimmt im Inneren eine Tragschraube auf, die an ihrem distalen Ende ein Knochengewinde aufweist. Ein Kopf der Tragschraube ist im Inneren der Traghülse frei beweglich in axialer Richtung bis der Kopf eine Ringstufe erreicht, die für den Kopf einen distalen Anschlag bildet. Die Ringstufe begrenzt ein weiteres Ausfahren der Tragschraube.

Das Dokument DE 692 11 561 T2 beschäftigt sich mit einer Vorrichtung zum Einsetzen eines Implantats. Das Implantat umfasst eine Hülse in die ein Bolzenteil eingeschraubt wird. In jeder Relativstellung sind Hülse und Bolzenteil gegen eine relative Axialverschiebung gesichert, indem ein Außengewinde am Bolzenteil und ein Innengewinde an der Hülse ineinandergreifen.

Dokument US 2004/0127900 A1 beschäftigt sich mit einer Knochenplattenanordnung, bei der eine Knochplatte mehrere Durchbrüche aufweist, in die beim Implantieren eine jeweilie Knochenschraube eingeschraubt wird.

Im Dokument US 2005/0143735 A1 ist eine Knochenschraubenanordnung beschrieben, bei der ein Schraubenschaft in ein Kompressionsteil eingeführt wird, derart, dass schließlich das Außengewinde am Schraubenschaft mit dem Innengewinde an dem Kompressionsteil zusammenwirkt.

Auch das Dokument US 2,801,631 offenbart eine Knochenschraubenanordnung, bei der ein Bolzenteil endseitig ein Knochengewinde aufweist. Im Gebrauch wird das Bolzenteil in eine Hülse eingeschraubt.

Im Dokument US 2010/10268285 A1 ist eine Knochenschraube mit einer Hülle und einem darin axial verschiebbar aufgenommenen Schaft offenbart. Der Schaft ist in der vollständig eingefahrenen Stellung gegen einen axialen Freilauf mittels einer am Schaft proximal vorgesehenen Endkappe gesperrt.

Im Dokument US 2005/0245933 A1 ist ein multikoaxiales Schraubensystem als Ankersystem für Knochen beschrieben. Eine innere Komponente der Schraubenanordnung ist in einer äußeren Komponente axial frei verschiebbar aufgenommen.

### Zusammenfassung

Aufgabe der Erfindung ist es, eine verbesserte Knochenschraubenanordnung variabler Länge zu schaffen, die sowohl eine Implantation als auch eine Explantation auf einfache und sichere Art und Weise ermöglicht.

Diese Aufgabe wird durch eine Knochenschraubenanordnung variabler Länge nach dem unabhängigen Anspruch 1 gelöst. Vorteilhafte Ausgestaltungen der Knochenschraubenanordnung sind Gegenstand von abhängigen Unteransprüchen.

Es ist eine Knochenschraubenanordnung variabler Länge mit einer Führungsschraube und einer Ankerschraube geschaffen. Die Führungsschraube verfügt über eine Führung, die sich im Inneren der Führungsschraube in axialer Richtung erstreckt. An der Ankerschraube ist ein Führungselement gebildet, das in der Führung aufgenommen ist, derart, dass eine von Führungsschraube und Ankerschraube gemeinsam gebildete Gesamtanordnungslänge veränderbar ist, indem das Führungselement, welches auch als Führungsabschnitt bezeichnet werden kann, in der Führung im Inneren der Führungsschraube in axialer Richtung verlagert wird. Hierdurch ändert sich dann die Überlappung zwischen Führungsschraube und Ankerschraube, die umso größer ist je weiter die Ankerschraube in der Führung der Führungsschraube aufgenommen ist. Je größer die Überlappung der beiden Schrauben ist, um kürzer ist die Gesamtlänge der Knochenschraubenanordnung.

Bei der die Gesamtschraubenlänge ändernden Verlagerung des Führungselementes in der Führung in axialer Richtung ist dieses in einem Abschnitt der Führung freilaufend aufgenommen, was in einer Ausführung bedeuten kann, dass die Ankerschraube in die Führungsschraube hinein geschoben und aus dieser heraus gezogen werden kann, ohne die beiden Schrauben hierbei relativ zueinander verdreht werden. Die freilaufende Aufnahme bedeutet insbesondere, dass das Führungselement axial wenigstens in eine Richtung verlagert werden kann, ohne dass die Führung dieser Axialverlagerung entgegensteht. Im Unterschied dazu ist das Führungselement zumindest in einer distalen Endstellung, in welcher die Ankerschraube vollständig ausgefahren ist, gegen einen axialen Freilauf gesperrt. Insbesondere ist eine freilaufende axiale Verschiebung des Führungselementes aus der distalen Endstellung unterbunden. Dies bedeutet nicht notwendigerweise, dass die Ankerschraube zur Verkürzung der Gesamtanordnungslänge gar nicht aus der Endstellung axial verlagerbar ist, eine solche Verlagerung ist jedoch vorzugsweise nur mittels Zwangsführung möglich, beispielweise dadurch, dass eine Drehbewegung von Führungsschraube oder Ankerschraube zwangsweise zur Axialverlagerung des Führungselementes in der Führung führt.

In dem weiteren Abschnitt ist in der Führung ein Innengewinde gebildet, welches zur Sperrung des axialen Freilaufs des Führungselementes mit einem am Führungselement gebildeten Außengewinde zusammenwirkt. Es kann vorgesehen sein, dass das Innengewinde und das Außengewinde mit im Wesentlichen gleicher axialer Länge hergestellt sind. Beim Drehen der Führungsschraube und / oder der Ankerschraube führt das Zusammenwirken von Innen- und Außengewinde dazu, dass zwangsweise die Gesamtanordnungslänge verändert wird, was einer Zwangsführung des Führungselementes der Ankerschraube in der Führung in der Führungsschraube entspricht. Das Zusammenwirken von Innen- und Außengewinde verhindert, dass die Relativstellung zwischen Ankerschraube und Führungsschraube in axialer Richtung ohne drehende Gewindebetätigung verändert werden kann, sodass das Führungselement in diesem Bereich der Führung, nämlich dem weiteren Führungsabschnitt, gerade nicht freilaufend in der Führung angeordnet ist. Vielmehr bedarf es einer Drehbewegung wenigstens der Führungsschraube oder wenigstens der Ankerschraube, um das Führungselement in der Führung axial zu verlagern und so die Gesamtlänge der Knochenschraubenanordnung zu verändern. Befindet sich das Innengewinde in zum Schraubenkopf der Führungsschraube distalen Endbereich der Führung, so gelangt das Führungselement mittels zwangsgeführter Verlagerung in die vollständig ausgefahrene Stellung, in welcher die Knochenschraubenanordnung ihre maximale Länge aufweist. Ein weitergehendes Drehen führt dann nicht mehr zur Längenveränderung der Knochenschraubenanordnung, sondern vielmehr zu einer zwangsweisen gemeinsamen Drehung von Führungsschraube und Ankerschraube, also der gesamten Anordnung. Dieses erleichtert beispielsweise ein Entfernen der Knochenschraubenanordnung aus dem Knochen oder einem Knochenverbund.

Vorzugsweise sind sowohl die Führungsschraube als auch die Ankerschraube jeweils als eine Knochenschraube gebildet, indem außenseitiges Knochenschraubgewinde vorgesehen ist.

In einer Ausführung ist das Führungselement hinsichtlich seiner äußeren Formgestaltung formschlüssig in der Führung aufgenommen.

In der Führung ist das Führungselement der Ankerschraube zwischen einer vollständig eingefahrenen und einer vollständig ausgefahrenen Stellung (distale Endstellung) verlagerbar, welche der kürzesten und der längsten Gesamtanordnungslänge der Knochenschraubenanordnung entsprechen. Ein Freilauf bedeutet hierbei zum Beispiel in einer Ausführung, dass bei den beiden Schrauben ihre axialen Relativlage zueinander (Überlappung in axialer Richtung) veränderbar ist, ohne das ein weiterer Aspekt der Relativlage verändert wird, insbesondere die relativen Drehstellungen zueinander (radiale Relativlage). Alternativ oder ergänzend ist eine freilaufende Änderung in axialer Richtung gegeben, wenn es hierzu nicht notwendig ist, an wenigstens einer der beiden schrauben ein Feststellmittel zu lösen.

Beispielsweise kann hierbei ein Gesamtführungsweg in axialer Richtung von etwa 20 mm vorgesehen sein. Die Gesamtanordnungslänge ist für unterschiedliche Knochenschraubenanordnungen variierbar, indem zum Beispiel Ankerschrauben unterschiedlicher Länge zum Einsatz kommen. Darüber hinaus kann für unterschiedliche Knochenschraubenanordnungen der axiale Verlagerungsweg in der Führung unterschiedlich lang sein.

Eine Weiterbildung sieht vor, dass die Führung einen weiteren Abschnitt aufweist, in welchem das Führungselement gegen den axialen Freilauf gesperrt ist. Bei dieser Ausführungs-form ist für das Führungselement entlang des weiteren Abschnitts eine freilaufende axiale Verschiebung blockiert. Der weitere Abschnitt der Führung kann sich ununterbrochen bis hin zur distalen Endstellung erstrecken.

Eine Ausführungsform sieht vor, dass das Führungselement am Schraubenkopf der Ankerschraube angeordnet ist. Es kann vorgesehen sein, dass der Schraubenkopf der Ankerschraube das Führungselement vollständig bildet. Es kann vorgesehen sein, dass sich das Außengewinde am Führungselement nur über einen Teil des Schraubenkopfes erstreckt.

Bevorzugt sieht eine Fortbildung vor, dass die Führungsschraube eine kopfseitige Öffnung aufweist, die mit der Führung in Verbindung steht. Die Öffnung ist beispielsweise als eine Bohrung gebildet, durch die von der Stirnseite her die Führung im Inneren der Führungsschraube zugänglich ist. Die Bohrung kann im Wesentlichen den gleichen Durchmesser wie die Führung im Inneren der Führungsschraube aufweisen, oder auch einen kleineren Durchmesser.

Bei einer Ausgestaltung kann vorgesehen sein, dass die kopfseitige Öffnung mit einem Deckel verschlossen ist, der lösbar montiert ist. Es kann vorgesehen sein, dass der Deckel als ein versenkter Deckel an der Führungsschraube aufgenommen ist.

Eine Weiterbildung kann vorsehen, dass der Deckel mittels eines Deckelschraubgewindes lösbar montiert ist. Es kann vorgesehen sein, dass der Deckel in eine stirnseitige Öffnung der Führungsschraube eingeschraubt ist. Vorzugsweise verfügt der Deckel über eine außenliegende Einsteckaufnahme, in die zum Drehen des Deckels ein Schraubdrehmittel eingesteckt werden kann. Im Querschnitt ist die Einsteckaufnahme beispielsweise als Sechskant hergestellt. In einer bevorzugten Ausführungsform ist die Einsteckaufnahme als ein Innensechskant gebildet, beispielsweise ein 5.0-Innensechskant. Mittels Einstecken eines Drehschraubmittels in die Einsteckaufnahme, beispielsweise eines Innbusschlüssels, kann der Deckel aus- und eingeschraubt werden, insbesondere zur Freigabe des Zugangs zu der Führung in der Führungsschraube.

Eine Weiterbildung sieht vor, dass das Deckelschraubgewinde gegenläufig ist zu dem Schraubgewinde ist, welches mit dem Innengewinde im Abschnitt der Führung und dem Außengewinde am Führungselement gebildet ist. Vorzugsweise ist das Deckelschraubgewinde als Linksgewinde ausgeführt.

Bei einer Ausgestaltung kann vorgesehen sein, dass der Schraubenkopf der Ankerschraube eine Einsteckaufnahme für ein Schraubendrehmittel aufweist, wobei die Einsteckaufnahme durch die kopfseitige Öffnung an der Führungsschraube zugänglich ist. Auch diese Einsteckaufnahme kann mit einem Innensechskant vorgesehen sein.

Eine Ausführungsform sieht vor, dass die Führungsschraube in einem zum Schraubenkopf distalen Endbereich mit einem Knochenschraubgewinde versehen ist.

Bevorzugt sieht eine Fortbildung vor, dass die Ankerschraube in einem zum Schraubenkopf distalen Endbereich mit einem Knochenschraubgewinde versehen ist.

### Beschreibung bevorzugter Ausführungsbeispiele

Die Erfindung wird im Folgenden anhand von bevorzugten Ausführungsbeispielen unter Bezugnahme auf Figuren einer Zeichnung näher erläutert. Hierbei zeigen:
- Fig. 1: eine perspektivische Darstellung einer Knochenschraubenanordnung,
- Fig. 2: eine schematische Darstellung der Knochenschraubenanordnung aus Fig. 1 im Längsschnitt in einer Ausgangsstellung,
- Fig. 3: eine schematische Darstellung der Knochenschraubenanordnung auf Fig. 1 im Längsschnitt in einer Stellung am Ende eines Knochenwachstums und
- Fig. 4: eine schematische Darstellung der Knochenschraubenanordnung aus Fig. 1 im Längsschnitt in einer Entnahmestellung.

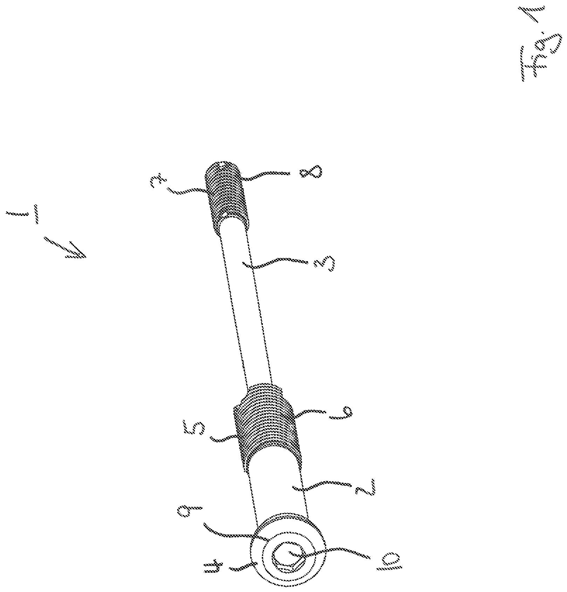

Fig. 1 zeigt eine perspektivische Darstellung einer Knochenschraubenanordnung 1 mit einer Führungsschraube 2 und einer Ankerschraube 3. Die Führungsschraube 2 ist in einem zum Schraubenkopf 4 der Führungsschraube 2 distalen Endbereich 5 mit einem Knochenschraubgewinde 6 versehen. Auch die Ankerschraube 3 verfügt in einem distalen Endbereich 7 über ein Knochenschraubgewinde 8.

In den Schraubenkopf 4 der Führungsschraube 2 ist stirnseitig ein Deckel 9 eingeschraubt. Der Deckel 9 verfügt über eine Einsteckaufnahme 10, die mit einem Innensechskant gebildet ist. In die Einsteckaufnahme 10 kann zum Betätigen des Deckels 9 ein Schraubdrehmittel eingesteckt werden, beispielsweise ein Inbusschlüssel.

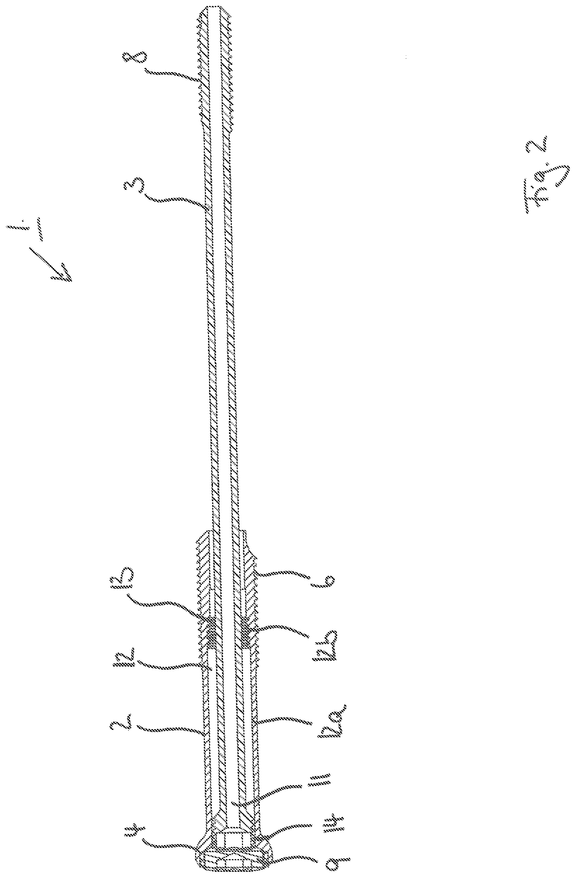

Fig. 2 zeigt eine schematische Darstellung der Knochenschraubenanordnung 1 im Längsschnitt. Ein an der Ankerschraube 3 gebildetes Führungselement 11, welches auch als Führungsabschnitt bezeichnet werden kann, ist in einer im Inneren der Führungsschraube 2 gebildeten Führung 12 in axialer Richtung verlagerbar aufgenommen. Die Führung 12 ist bei dem dargestellten Führungsbeispiel als sich in axialer Richtung erstreckender Hohlraum ausgeführt, in welchem die Ankerschraube 3 mit dem Führungselement 11 geführt ist, welches vorzugsweise formschlüssig hierin aufgenommen ist. In einem Abschnitt 12a der Führung 12 ist das Führungselement 11 freilaufend axial verlagerbar, derart, dass das Führungselement 11 im Abschnitt 12a frei gleiten kann. Auch können die Ankerschraube 3 und somit das hieran gebildete Führungselement 11 im Bereich des Abschnittes 12a frei auf der Stelle gedreht werden. Die Führung 12 bildet im Abschnitt 12a eine rohrförmige Aufnahme mit glatter Oberfläche auf der das Führungselement 11 frei bewegbar ist.

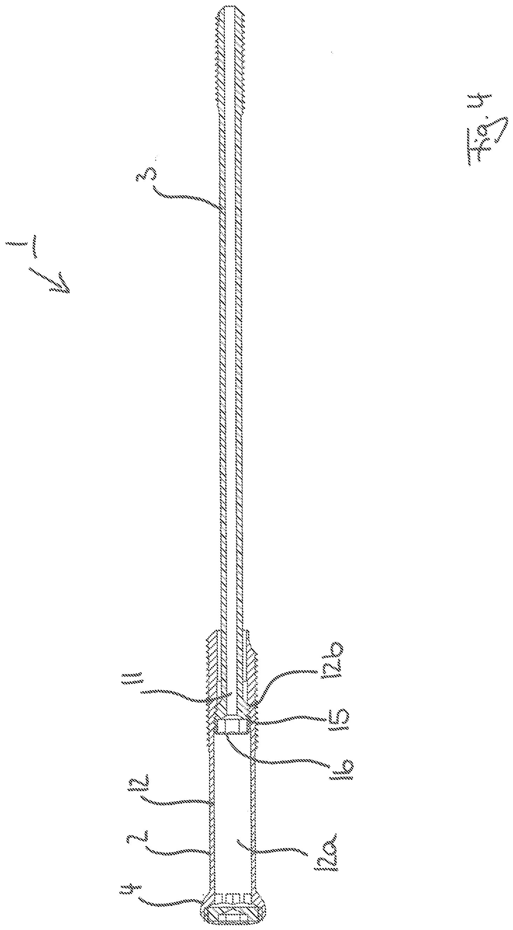

In ihrer Relativstellung zu der Führungsschraube 2 ist die Ankerschraube 3 zwischen der in Fig. 2 gezeigten vollständig eingefahrenen Stellung und der in Fig. 4 gezeigten vollständig ausgefahrenen Stellung verlagerbar, sodass auf dieses Weise eine Gesamtlänge der Knochenschraubenanordnung veränderbar ist. Die Gesamtanordnungslänge ist bei der Stellung in Fig. 2 minimal und in der Ausführung nach Fig. 4 maximal.

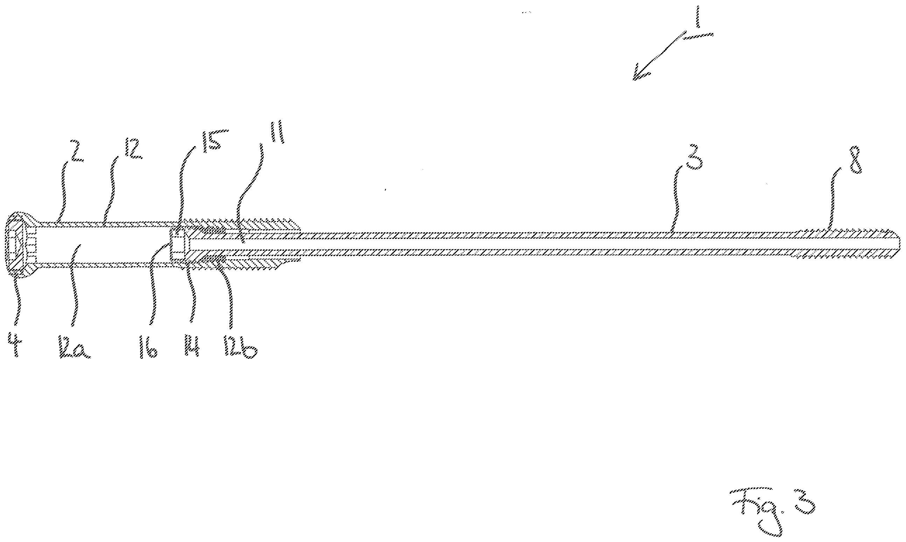

In dem Abschnitt 12a ist das Führungselement 11 in der Führung 12 freilaufend in axialer Richtung verlagerbar. Bei der gezeigten Ausführungsform gleitet das Führungselement 11 hierbei frei auf der Wandung der Führung 12 bis in die in Fig. 3 gezeigte Stellung. Diese zeigt die Knochenschraubenanordnung 1 am Ende eines Prozesses, in dem sich die Gesamtlänge der Knochenschraubenanordnung 1 verlängert, beispielsweise aufgrund eines Knochenwachstums im Verlauf eines Heilungsprozesses, welcher durch die Knochenschraubenanordnung 1 unterstützt wird. Das Knochenwachstum kann sich hierbei frei entfalten, solange dies die freilaufende Axialverlagerung des Führungselementes 11 in der Führung 12 zulässt.

Dieser Freilauf wird dann in der in Fig. 3 gezeigten Stellung gestoppt, wo sich das Führungselement 11 dann am distalen Ende des Abschnittes 12a befindet. Eine weitere Verlängerung der Gesamtlänge der Knochenschraubenanordnung 1 ist dann nur noch mittels einer Zwangsführung des Führungselementes 11 möglich. Hierbei wirken ein Innengewinde 13, das in einem Abschnitt 12b auf der inneren Oberfläche der Führung 12 gebildet ist, sowie ein Außengewinde 14 auf dem Führungselement 11 zusammen. Mittels Drehen der Führungsschraube 2 wird so das Führungselement 11 aus der in Fig. 3 gezeigten Stellung in die in Fig. 4 gezeigte Stellung verlagert, welche einer distalen Endstellung entspricht, in der die Knochenschraubenanordnung 1 ihre maximale Gesamtlänge aufweist.

Ist die Ankerschraube 3 in einem Knochen verankert, so führt das Drehen der Führungsschraube 2 und das hierbei bewirkte Zusammenwirken von Innengewinde 13 und Außengewinde 14 vorzugsweise zu einem teilweisen Herausschrauben der Führungsschraube 2 aus dem Knochen, da die Führungsschraube 2 zwangsweise in eine Richtung vom distalen Ende der Ankerschraube 3 weg bewegt wird. Ein weiteres Drehen der Führungsschraube 2 nach dem Erreichen der in Fig. 4 gezeigten Relativstellung zwischen Führungsschraube 2 und Ankerschraube 3 führt dann dazu, dass die Knochenschraubenanordnung 1 in ihrer Gesamtheit aus den Knochen heraus gedreht wird. Hierbei sind Führungsschraube 2 und Ankerschraube 3 aufgrund des Zusammenwirkens von Innengewinde 13 und Außengewinde 14 zueinander fixiert. Innen- und Außengewinde 13, 14 wirken als Kontergewinde, was ein stabiles Herausdrehen der Knochenschraubenanordnung 1 zur Explantation unterstützt. Ein Schraubendrehmittel greift hierbei in die Einsteckaufnahme 10 am Deckel 9 ein.

Es ist vorgesehen, dass ein Deckelschraubgewinde, welches zur Montage des Deckels 9 an der Führungsschraube 2 dient, gegenläufig zu dem mit dem Innen- und Außengewinde 13, 14 gebildeten Gewinde ist, sodass mittels Drehen am Deckel 9 das Ausbilden der in Fig. 4 gezeigten Relativstellung von Führungsschraube 2 und Ankerschraube 3 bewirkt wird und anschließend der Gesamtverbund herausgedreht werden kann aus dem Knochen.

Aus den Fig. 2 bis 4 ergibt sich, dass bei der gezeigten Ausführungsform das Führungselement 11 am Schraubenkopf 15 der Ankerschraube 3 gebildet ist. Der Schraubenkopf 15 verfügt seinerseits über eine Einsteckaufnahme 16, die bei der gezeigten Ausführungsform wieder mit einem Innensechskant versehen ist, vorzugsweise in der Größe M5. Bei herausgeschraubtem Deckel 9 ist die Einsteckaufnahme 16 in der Führung 12 zugänglich, insbesondere beim Eindrehen der Ankerschraube 3 beim Implantieren der Knochenschraubenanordnung 1.

Die in der vorstehenden Beschreibung, den Ansprüchen und der Zeichnung offenbarten Merkmale können sowohl einzeln als auch in beliebiger Kombination für die Verwirklichung von verschiedenen Ausführungen von Bedeutung sein.

## Patentansprüche

1. Knochenschraubenanordnung (1) variabler Länge, mit:
- einer Führungsschraube (2), welche mit einer Führung (12) gebildet ist, die sich im Inneren der Führungsschraube (2) in axialer Richtung erstreckt, und
- einer Ankerschraube (3), welche ein Führungselement (11) aufweist, das in der Führung (12) aufgenommen ist, derart, dass eine von Führungsschraube (2) und Ankerschraube (3) gemeinsam gebildete Anordnungslänge veränderbar ist, indem das Führungselement (11) in der Führung (12) im Inneren der Führungsschraube (2) in axialer Richtung verlagert wird,
wobei das Führungselement (11) bei der die Anordnungslänge ändernden Verlagerung in einem Abschnitt (12a) der Führung (12) freilaufend aufgenommen und zumindest in einer distalen Endstellung, in welcher die Ankerschraube (3) vollständig ausgefahren ist, gegen einen axialen Freilauf gesperrt ist, wobei die Führung (12) einen weiteren Abschnitt (12b) aufweist, in welchem das Führungselement (11) gegen den axialen Freilauf gesperrt ist, **dadurch gekennzeichnet, dass** in dem weiteren Abschnitt (12b) in der Führung (12) ein Innengewinde (13) gebildet ist, welches zur Sperrung des axialen Freilaufs des Führungselementes (11) mit einem am Führungselement (11) gebildeten Außengewinde (14) zusammenwirkt.

2. Knochenschraubenanordnung (1) nach Anspruch 1, **dadurch gekennzeichnet, dass** das Führungselement (11) am Schraubenkopf (15) der Ankerschraube (3) angeordnet ist.

3. Knochenschraubenanordnung (1) nach mindestens einem der vorangehenden Ansprüche, **dadurch gekennzeichnet, dass** die Führungsschraube (2) eine kopfseitige Öffnung aufweist, die mit der Führung (12) in Verbindung steht.

4. Knochenschraubenanordnung (1) nach Anspruch 3, **dadurch gekennzeichnet, dass** die kopfseitige Öffnung mit einem Deckel (9) verschlossen ist, der lösbar montiert ist.

5. Knochenschraubenanordnung (1) nach Anspruch 4, **dadurch gekennzeichnet, dass** der Deckel (9) mittels eines Deckelschraubgewindes lösbar montiert ist.

6. Knochenschraubenanordnung (1) nach Anspruch 5, **dadurch gekennzeichnet, dass** das Deckelschraubgewinde gegenläufig zu dem Schraubgewinde ist, welches mit dem Innengewinde (13) im Abschnitt (12a) der Führung (12) und dem Außengewinde (14) am Führungselement (11) gebildet ist.

7. Knochenschraubenanordnung (1) nach mindestens einem der vorangehenden Ansprüche, soweit auf Anspruch 3 rückbezogen, **dadurch gekennzeichnet, dass** der Schraubenkopf (15) der Ankerschraube (3) eine Einsteckaufnahme (16) für ein Schraubdrehmittel aufweist, wobei die Einsteckaufnahme (16) durch die kopfseitige Öffnung an der Führungsschraube (2) zugänglich ist.

8. Knochenschraubenanordnung (1) nach mindestens einem der vorangehenden Ansprüche, **dadurch gekennzeichnet, dass** die Führungsschraube (2) in einem zum Schraubenkopf (4) distalen Endbereich (5) mit einem Knochenschraubgewinde (6) versehen ist.

9. Knochenschraubenanordnung (1) nach mindestens einem der vorangehenden Ansprüche, **dadurch gekennzeichnet, dass** die Ankerschraube (3) in einem zum Schraubenkopf (15) distalen Endbereich (7) mit einem Knochenschraubgewinde (8) versehen ist.

## Claims

1. A bone screw arrangement (1) with variable length, comprising:
- a guide screw (2), which is formed with a guide (12), which extends in axial direction in the interior of the guide screw (2), and
- an anchor screw (3), which has a guide element (11), which is received in the guide (12), such that an arrangement length formed jointly by guide screw (2) and anchor screw (3) is able to be altered by the guide element (11) being displaced in axial direction in the guide (12) in the interior of the guide screw (2),
wherein the guide element (11), on the displacement altering the arrangement length, is received in a free-running manner in a portion (12a) of the guide (12) and is blocked from an axial free-running at least in a distal end position in which the anchor screw (3) is fully extended, wherein the guide (12) has a further portion (12b), in which the guide element (11) is blocked from axial free-running, **characterized in that** in the further portion (12b) in the guide (12) an internal thread (13) is formed which, for blocking the axial free-running of the guide element (11), cooperates with an external thread (14) formed on the guide element (11).

2. The bone screw arrangement (1) according to Claim 1, **characterized in that** the guide element (11) is arranged on the screw head (15) of the anchor screw (3).

3. The bone screw arrangement (1) according to at least one of the preceding claims, **characterized in that** the guide screw (2) has a head-side opening which is connected with the guide (12).

4. The bone screw arrangement (1) according to Claim 3, **characterized in that** the head-side opening is closed by a cover (9) which is detachably mounted.

5. The bone screw arrangement (1) according to Claim 4, **characterized in that** the cover (9) is detachably mounted by means of a cover screw thread.

6. The bone screw arrangement (1) according to Claim 5, **characterized in that** the cover screw thread runs in the opposite direction to the screw thread, which is formed with the internal thread (13) in the portion (12a) of the guide (12) and with the external thread (14) on the guide element (11).

7. The bone screw arrangement (1) according to at least one of the preceding claims, in so far as relating to Claim 3, **characterized in that** the screw head (15) of the anchor screw (3) has an insertion mount (16) for a screw turning means, wherein the insertion mount (16) is accessible through the head-side opening on the guide screw (2).

8. The bone screw arrangement (1) according to at least one of the preceding claims, **characterized in that** the guide screw (2) is provided with a bone screw thread (6) in an end region (5) distal to the screw head (4).

9. The bone screw arrangement (1) according to at least one of the preceding claims, **characterized in that** the anchor screw (3) is provided with a bone screw thread (8) in an end region (7) distal to the screw head (15).

## Revendications

1. Agencement de vis à os (1) de longueur variable, comprenant :
- une vis de guidage (2) qui est formée avec un guide (12) qui s'étend axialement à l'intérieur de la vis de guidage (2), et
- une vis d'ancrage (3) qui présente un élément de guidage (11) qui est reçu dans le guide (12) de telle sorte qu'une longueur d'agencement formée de façon commune par la vis de guidage (2) et la vis d'ancrage (3) est modifiable en ce que l'élément de guidage (11) dans le guide (12) est disposé axialement à l'intérieur de la vis de guidage (2),
dans lequel l'élément de guidage (11), dans la disposition modifiant la longueur d'agencement, est reçu en roue libre dans un tronçon (12a) du guide (12) et est bloqué contre une marche en roue libre axiale dans au moins une position d'extrémité distale dans laquelle la vis d'ancrage (3) est complètement sortie, dans lequel le guide (12) présente un tronçon supplémentaire (12b) dans lequel l'élément de guidage (11) est bloqué contre la marche en roue libre axiale, **caractérisé en ce qu'**un filetage femelle (13) est formé dans le tronçon supplémentaire (12b) dans le guide (12), lequel coopère avec un filetage mâle (14) formé sur l'élément de guidage (11) pour bloquer la marche en roue libre axiale de l'élément de guidage (11).

2. Agencement de vis à os (1) selon la revendication 1, **caractérisé en ce que** l'élément de guidage (11) est disposé sur la tête (15) de la vis d'ancrage (3).

3. Agencement de vis à os (1) selon au moins l'une quelconque des revendications précédentes, **caractérisé en ce que** la vis de guidage (2) présente une ouverture côté tête qui est en liaison avec le guide (12).

4. Agencement de vis à os (1) selon la revendication 3, **caractérisé en ce que** l'ouverture côté tête est fermée par un couvercle (9) qui est monté amovible.

5. Agencement de vis à os (1) selon la revendication 4, **caractérisé en ce que** le couvercle (9) est monté amovible au moyen d'un filetage vissé de couvercle.

6. Agencement de vis à os (1) selon la revendication 5, **caractérisé en ce que** le filetage vissé de couvercle est en sens opposé du filetage vissé qui est formé par le filetage femelle (13) dans le tronçon (12a) du guide (12) et le filetage mâle (14) sur l'élément de guidage (11).

7. Agencement de vis à os (1) selon au moins l'une quelconque des revendications précédentes, dans la mesure où l'on se rapporte à la revendication 3, **caractérisé en ce que** la tête de vis (15) de la vis d'ancrage (3) présente une réception d'insertion (16) pour un moyen de rotation de vis, dans lequel la réception d'insertion (16) est accessible par l'ouverture côté tête sur la vis de guidage (2).

8. Agencement de vis à os (1) selon au moins l'une quelconque des revendications précédentes, **caractérisé en ce que** la vis de guidage (2) est dotée d'un filetage de vis à os (6) dans une zone d'extrémité (5) distale par rapport à la tête de vis (4).

9. Agencement de vis à os (1) selon au moins l'une quelconque des revendications précédentes, **caractérisé en ce que** la vis d'ancrage (3) est dotée d'un filetage de vis à os (8) dans une zone d'extrémité (7) distale par rapport à la tête de vis (15).
